# EUROPÄISCHE PATENTSCHRIFT

(11) **EP 1 128 724 B1**
(45) Veröffentlichungstag und Bekanntmachung des Hinweises auf die Patenterteilung: **27.11.2002**
(21) Anmeldenummer: 99957981.6
(22) Anmeldetag: 02.11.1999
(51) Int. Cl.: A01N 25/34, A01N 25/10, A61L 29/16, A61L 29/06

(54) **WIRKSTOFFHALTIGE POLYETHERBLOCKAMIDE**
POLYETHER BLOCK AMIDES CONTAINING ACTIVE SUBSTANCES
AMIDES DE POLYETHER SEQUENCE CONTENANT DES SUBSTANCES ACTIVES

(30) Priorität: 12.11.1998 DE 19852190
(43) Veröffentlichungstag der Anmeldung: 05.09.2001
(73) Patentinhaber: Bayer Aktiengesellschaft, 51368 Leverkusen (DE)
(72) Erfinder: ALBERS, Reinhard, D-51375 Leverkusen (DE); DUJARDIN, Ralf, Novi, MI 48374 (US); PUDLEINER, Heinz, D-47800 Krefeld (DE); SIMON, Joachim, D-40589 Düsseldorf (DE); WAGNER, Joachim, D-51061 Köln (DE); HOBLER, Hartwin, D-42111 Wuppertal (DE)
(86) Internationale Anmeldenummer: EP9908353
(87) Internationale Veröffentlichungsnummer: WO00028814

(56) Entgegenhaltungen:
- WO-A-96/22114
- FR-A- 2 273 021
- US-A- 5 165 952
- US-A- 5 556 383
- PATENT ABSTRACTS OF JAPAN vol. 1997, no. 02, 28. Februar 1997 (1997-02-28) & JP 08 280790 A (OTSUKA PHARMACEUT FACTORY INC), 29. Oktober 1996 (1996-10-29) -& DATABASE WPI Week 9702 Derwent Publications Ltd., London, GB; AN 1997-015196 XP002135906 & JP 08 280790 A -& JP 08 280790 A (OTSUKA PHAMRMACEUT FACTORY INC)
- SCHIERHOLZ J M ET AL: "Controlled release of antibiotics from biomedical polyurethanes: morphological and structural features" BIOMATERIALS,GB,ELSEVIER SCIENCE PUBLISHERS BV., BARKING, Bd. 18, Nr. 12, 1. Januar 1997 (1997-01-01), Seiten 839-844, XP004064466 ISSN: 0142-9612

## Beschreibung

Die Erfindung betrifft antimikrobiell wirksame Stoffe in homogener Verteilung enthaltende Polyetherblockamide (PEBA), Verfahren zu ihrer Herstellung und deren Verwendung in medizinischen Artikeln.

Vaskuläre, Katheter-assoziierte Infektionen stellen eine wichtige Ursache bei der Morbidität und Mortalität von Patienten der Intensivstation dar. Neuere Studien belegen, daß in diesem Patientenkollektiv bis 16 % der Katheterträger eine Katheterinduzierte Sepsis erleiden. Ca. 2 % dieser Patienten zeigen ernsthafte klinische Komplikationen, insbesondere einen septischen Schock oder ein akutes Organversagen. In der Zukunft ist mit einer weiter steigenden Inzidenz von Katheterinfektionen zu rechnen, da Katheter in der modernen Intensivtherapie zunehmend bei invasiven Monitoring- bzw. Behandlungsstrategien, wie z.B. der kontinuierlichen Hämofiltration Anwendung finden.

Zahlreiche Studien haben ergeben, daß koagulase-negative Staphylococcen, der transiente Keim Staphylococcus aureus und verschiedene Candida Spezies die Hauptverursacher von Katheter-assoziierten Infektionen sind. Diese ubiquitär auf der Haut vorhandenen Mikroorganismen durchdringen bei der Applikation des Katheters die physiologische Hautbarriere und gelangen so in den subkutanen Bereich und letztendlich in die Blutbahn. Die Adhäsion der Bakterien auf der Kunststoffoberfläche wird als essentieller Schritt bei der Pathogenese von Fremdkörperinfektionen betrachtet. Nach der Adhäsion der Hautkeime an der Polymeroberfläche folgt die Proliferation der Bakterien mit der Besiedlung des Polymers. Damit einher geht die Produktion eines Biofilms durch bakterielle Exkretion von extrazellulärer Glykocalix. Der Biofilm unterstützt die Adhäsion der Erreger und schützt sie vor der körpereigenen Immunabwehr. Zudem bildet der Biofilm eine für viele Antibiotika undurchdringliche Barriere. Nach verstärkter Proliferation der pathogenen Keime an der Polymeroberfläche, kann es schließlich zu einer septischen Bakteriämie kommen. Zur Therapie derartiger Infektionen ist eine Entfernung des infizierten Katheters nötig, da eine Chemotherapie mit Antibiotika unphysiologisch hohe Dosen verlangen würde.

Die Verwendung von zentralvenösen Kathetern beinhaltet daher nicht nur ein hohes Infektionsrisiko für die Patienten, sondern verursacht auch enorme therapeutische Folgekosten.

Durch prophylaktische Maßnahmen, wie z. B. hygienische Maßnahmen oder routinemäßige endoluminale Antibiotikaapplikationen kann diese Problematik nur teilweise gelöst werden. Eine rationale Strategie zur Prävention von Polymer-assoziierten Infektionen besteht in der Modifizierung der verwendeten polymeren Materialien. Ziel dieser Modifizierung muß die Hemmung der Bakterienadhäsion bzw. der Proliferation bereits adhärierter Bakterien sein, um somit kausal Fremdkörperinfektionen zu vermeiden. Dies kann z.B. durch Inkorporierung einer geeigneten antimikrobiell wirksamen Substanz in die Polymermatrix (z.B. Antibiotika) gelingen, vorausgesetzt, daß der eingearbeitete Wirkstoff auch aus der Polymermatrix herausdiffundieren kann. In diesem Fall kann die Freisetzung des Wirkstoffs auf einen längeren Zeitraum ausgedehnt werden, damit für einen entsprechend längeren Zeitraum die Bakterienadhäsion bzw. Proliferation auf dem Polymer verhindert wird.

Methoden zur Herstellung antimikrobiell ausgestatteter Polymere für medizinische Anwendungen sind bereits bekannt. In den zahlreich beschriebenen Verfahren erfolgt der Zusatz des Wirkstoffs durch folgende Techniken:
a) Adsorption auf der Polymeroberfläche (passiv oder via Surfactants)
b) Einbringen in eine Polymerbeschichtung, die auf der Oberfläche eines Formkörpers appliziert wird
c) Inkorporierung in die bulk-Phase des polymeren Trägermaterials
d) Kovalente Bindung an der Polymeroberfäche

Aus JP-A 08 280 790 gehen Polymermatrices hervor, die antibakterielle Stoffe in homogener Verteilung enthalten. Als Polymermatrices werden Ethylen-Vinyl-Alkohol-Copolymere und Polyetherpolymere eingesetzt. Als antibakterielle Stoffe finden Chinoline Anwendung. Die Polymermatrices werden in medizinischen Artikeln, wie z.B. Kathetern eingesetzt.

Aus DE-A-41 43 239 geht beispielsweise ein Verfahren hervor, Wirkstoffe in die äußere Schicht medizinischer Artikel einzubringen (Imprägnierung). Dabei wird die implantierbare Vorrichtung aus polymerem Material in einem geeigneten Lösungsmittel gequollen. Die Polymermatrix wird dabei so verändert, dass ein pharmazeutischer Wirkstoff bzw. eine Wirkstoffkombination in das polymere Material des Implantats eindringen kann. Nach Entfernen des Lösungsmittels wird der Wirkstoff in der Polymermatrix eingeschlossen. Nach Kontakt mit physiologischen Medium wird der in der implantierbaren Vorrichtung enthaltene Wirkstoff durch Diffusion wieder freigesetzt. Das Freisetzungsprofil kann dabei durch die Wahl des Lösungsmittels und durch Variation der experimentellen Bedingungen eingestellt werden.

Polymermaterialien für medizinische Anwendungen, die wirkstoffhaltige Beschichtungen aufweisen, werden beispielsweise in EP-A 328 421 erwähnt. Beschrieben werden Verfahren zur Herstellung der antimikrobiell wirksamen Beschichtungen sowie Methoden zur Applikation auf die Oberflächen von medizinischen Devices. Die Beschichtungen bestehen aus einer Polymermatrix, insbesondere aus Polyurethanen, Silikonen oder bioabbaubaren Polymeren, und einer antimikrobiell wirksamen Substanz, vorzugsweise einer synergistischen Kombination eines Silbersalzes mit Chlorhexidin oder einem Antibiotikum.

Allen erwähnten Verfahren ist gemeinsam, dass das Ausrüsten des medizinischen Arbeitsmittels mit einer antimikrobiell wirksamen Substanz einen zusätzlichen Arbeitsschritt erforderlich macht, nämlich entweder eine Vorbehandlung des Polymermaterials vor der Verarbeitung oder eine Nachbehandlung der hergestellten Formkörper. Dies verursacht zusätzliche Kosten und bringt einen erhöhten Zeitaufwand bei der Produktion mit sich. Eine weitere Problematik der Verfahren besteht in der Verwendung von organischen Lösungsmitteln, die meist nicht restlos aus dem Material entfernt werden können.

Aufgabe der Erfindung war es, neue Polymermaterialien bereitzustellen, die zur Herstellung von medizinischen Formkörpern für Kurzzeitimplantate, insbesondere Kathetern, geeignet sind, und für einen längeren Zeitraum (2-4 Wochen) effizient eine Oberflächenbesiedlung durch Keime verhindern.

Es wurden nun antimikrobiell wirksame Stoffe in homogener Verteilung enthaltende Polyetherblockamide gefunden, die über einen längeren Zeitraum eine die Besiedelung mit Keimen unterbindende Konzentration eines antimikrobiell wirksamen Stoffes an der Oberfläche freisetzen.

Gegenstand der Erfindung sind somit Polyetherblockamide, die aus Polymerketten bestehen, die aus wiederkehrenden Einheiten entsprechend der Formel I aufgebaut sind 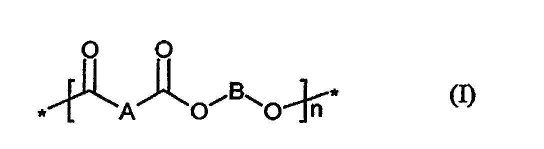 in welcher
- A: die von einem Polyamid mit 2 Carboxylendgruppen durch Verlust der letzteren abgeleitete Polyamidkette ist und
- B: die von einem Polyoxyalkylenglycol mit endständigen OH-Gruppen durch Verlust der letzteren abgeleitete Polyoxyalkylenglycolkette ist und
- n: die Zahl der die Polymerkette bildenden Einheiten ist. Als Endgruppen stehen dabei bevorzugt OH-Gruppen oder Reste von Verbindungen, die die Polymerisation abbrechen,
und die einen antimikrobiell wirksamen Stoff in homogener Verteilung enthalten aus der Gruppe bestehend aus
a) Nalixidinsäure, Pipemidsäure, Cinoxacin, Ciprofloxacin, Norfloxacin, Ofloxacin, Pefloxacin, Enoxacin,
b) Aminoglycoside,
c) Makrolide,
d) Polypeptide,
e) Lincomycine,
f) antimycobakterielle Mittel,
g) Fusidinsäure,
wobei die Kombination von Polyetherblockamid und antimikrobiell wirksamen Stoff eine Grenzflächenenergie von 0,5 bis 30 mN/m aufweist, zur Herstellung von medizinischen Formkörpern für Kurzzeitimplantate, die für einen längeren Zeitraum eine Oberflächenbesiedlung durch Keime verhindern.

Als antimikrobiell wirksame Substanzen kommen prinzipiell alle Wirkstoffe in Betracht, die ein breites Wirkungsspektrum gegen die bei Polymer-assoziierten Infektionen involvierten pathogenen Mikroorganismen aufweisen, insbesondere gegen koagulase-negative Staphylokokken, Staphylokokkus aureus und Candida-Spezies. Die antimikrobiell wirksamen Substanzen können erfindungsgemäß auch als Wirkstoffkombinationen in den Formkörpern verwendet werden, sofern sich ihre Wirkungen nicht antagonisieren.

Die verwendete antimikrobiell wirksame Substanz muss eine ausreichende (chemische) Stabilität in der Polymermatrix besitzen. Zudem darf die mikrobiologische Aktivität des Wirkstoffs in der polymeren Matrix und unter den Verfahrensbedingungen der Einarbeitung nicht beeinträchtigt werden, der Wirkstoff muss also bei den für die thermoplastische Verarbeitung des polymeren Materials erforderlichen Temperaturen von 150 bis 200°C stabil sein.

Die Inkorporierung der pharmazeutisch wirksamen Substanz sollte weder die Biokompatibilität der Polymeroberfäche noch andere polymer-spezifische Eigenschaften des polymeren Materials (Elastizität, Reißfestigkeit etc.) beeinträchtigen.

Geeignete antibiotisch wirksame Substanzen sind beispielsweise:
- Aminoglycoside, wie z.B. Gentamycin, Kanamycin, Amikacin, Sisomycin, Tobramycin, Netilmicin, vorzugsweise Gentamycin und Kanamycin,
- Makrolide wie z.B. Erythromycin, Clarithromycin und Azithromycin
- Polypeptide, wie z.B. Bacitracin, Mupirocin, Thyrothricin (Kombination von Gramicidin und Tyrocidin,
- Lincomycine, wie z.B. Lincomycin und Clindamycin
- antimycobakterielle Mittel, wie z.B. Rifampicin.

Die antimikrobiell wirksame Substanz kann auch ein Antiseptikum oder ein Desinfektionsmittel sein, solange die verwendete Substanz eine ausreichende Wirksamkeit gegen die infektionsverursachenden Spezies besitzt.

Darüber hinaus können Substanzen (pro-drugs) eingesetzt werden, die nach Einfluss mikrobieller Tätigkeit eine antimikrobiell wirksame Substanz freisetzen.

Die Wirkstoffe werden vorzugsweise in einer ihrer antimikrobiellen Aktivität entsprechenden Konzentration eingearbeitet. Besonders bevorzugt werden die Wirkstoffe in einem Konzentrationsbereich von 0,01 bis 10,0 Gew.-% verwendet.

Die Dicarbonsäurepolyamide mit den endständigen Carboxylgruppen werden auf bekannte Weise erhalten, so z.B. durch Polykondensation eines oder mehrerer Lactame oder/und einer oder mehrerer Aminosäure, ferner durch Polykondensation einer Dicarbonsäure mit einem Diamin, jeweils in Gegenwart eines Überschusses einer organischen Dicarbonsäure, vorzugsweise mit endständigen Carboxylgruppen. Diese Carbonsäuren werden während der Polykondensation Bestandteil der Polyamidkette und lagern sich insbesondere an den Ende derselben an, wodurch man ein α,ω-dicarbonsaures Polyamid erhält. Ferner wirkt die Dicarbonsäure als Kettenabbruchmittel, weshalb sie auch im Überschuss eingesetzt wird.

Das Polyamid kann erhalten werden, ausgehend von Lactamen und/oder Aminosäuren mit einer Kohlenwasserstoffkette bestehend aus 4-14 C-Atomen, wie z.B. von Caprolactam, Onantholactam, Dekanolactam, Undekanolactam, Dodekalactam, 11-Amino-undekano oder 12-Aminododekansäure.

Als Beispiele für Polyamide, wie sie durch Polykondensation einer Dicarbonsäure mit einem Diamin entstehen, können genannt werden die Kondensationsprodukte aus Hexamethylendiamin mit Adipin-, Azelain-, Sebacin-, und 1,12-Dodecandisäure, sowie die Kondensationsprodukte aus Nonamethylendiamin und Adipinsäure.

Bei den zur Synthese des Polyamids, das heißt, einerseits zur Fixierung jeweils einer Carboxylgruppe an jedem Ende der Polyamidkette und andererseits als Kettenabbruchmittel verwendeten Dicarbonsäure kommen solche mit 4-20 C-Atomen in Frage, insbesondere Alkandisäuren, wie Bernstein-, Adipin-, Kork-, Azelain-, Sebacin-, Undekandi- oder Dodekandisäure, ferner cycloaliphatische oder aromatische Dicarbonsäure, wie Terephthal- oder Isophthal- oder Cyclohexan-1,4-dicarbonsäure.

Die endständigen OH-Gruppen aufweisenden Polyoxyalkylenglycole sind unverzweigt oder verzweigt und weisen einen Alkylenrest mit mindestens 2 C-Atomen auf. Insbesondere sind dies Polyoxyethylen-, Polyoxypropylen- und Polyoxytetramethylenglycol, sowie Copolymerisate davon.

Das Durchschnittsmolekulargewicht dieser OH-Gruppen-terminierten Polyoxyalkylenglycole kann sich in einem großen Bereich bewegen, vorteilhaft liegt es zwischen 100 und 6000, insbesondere zwischen 200 und 3000.

Der Gewichtsanteil des Polyoxyalkylenglycols, bezogen auf das Gesamtgewicht des zur Herstellung des PEBA-Polymeren verwendeten Polyoxyalkylenglycols und Dicarbonsäurepolyamids beträgt 5-85 % vorzugsweise 10-50 %.

Verfahren zur Synthese derartiger PEBA-Polymerer sind aus FR-A 2 273 021, DE-OS 28 02 989, DE-OS 28 37 687, DE-OS 25 23 991, EP-A 095 893, DE-OS 27 12 987 beziehungsweise DE-OS 27 16 004 bekannt.

Erfindungsgemäß bevorzugt geeignet sind solche PEBA-Polymere, die im Gegensatz zu den vorher beschriebenen, statistisch aufgebaut sind. Zur Herstellung dieser Polymere wird ein Gemisch aus
1. einer oder mehrerer polyamidbildender Verbindungen aus der Gruppe der α,ω-Aminocarbonsäuren beziehungsweise Lactame mit mindestens 10 Kohlenstoffatomen,
2. einem α,ω-Dihydroxy-Polyoxyalkylenglycol,
3. mindestens einer organischen Dicarbonsäure
in einem Gewichtsverhältnis von 1:(2+3) zwischen 30:70 und 98:2, wobei in (2+3) Hydroxyl- und Carbonylgruppen in äquivalenten Mengen vorliegen, in Gegenwart von 2 bis 30 Gewichtsprozent Wasser, bezogen auf die polyamidbildenden Verbindungen der Gruppe 1, unter dem sich einstellenden Eigendruck auf Temperaturen zwischen 23°C und 30°C erhitzt und anschließend nach Entfernen des Wassers unter Ausschluss von Sauerstoff bei Normaldruck oder unter vermindertem Druck bei 250 bis 280°C weiterbehandelt werden.

Solche bevorzugt geeignete PEBA-Polymere sind z.B. in DE-OS 27 12 987 beschrieben.

Geeignete PEBA-Polymere sind z.B. unter den Handelsnamen PEBAX® der Fa. Atochem, Vestamid® der Fa. Hüls AG, Grilamid® der Fa. EMS-Chemie und Kellaflex® der Fa. DSM erhältlich.

Die beschriebenen PEBA-Polymere sind durch ein sehr gutes mechanisches Eigenschaftsprofil gekennzeichnet. Die Shore Härte kann je nach Zusammensetzung der PEBA-Polymere über einen weiten Bereich (Shore 70 A - Shore 60 D) variiert werden. Die weicheren PEBA-Einstellungen zeichnen'sich gegenüber vergleichbaren thermoplastischen Polyurethanen (TPU) durch eine ausgezeichnete Verarbeitbarkeit aus. Durch den Einsatz weicher Polymermaterialien kann das Risiko einer Verletzung von Gefäßwänden deutlich reduziert werden. Darüber hinaus liegen die Moduli der PEBA-Polymere im Vergleich zu thermoplastischen Polyurethanen bei gleicher Härte auf höherem Niveau.

Aufgrund dieser spezifischen Eigenschaften sind die PEBA-Polymere für die Herstellung von medizinischen Devices, insbesondere von Kathetern, geeignet.

Die erfindungsgemäßen wirkstoffhaltigen Polyetherblockamide können weiterhin die für Kunststoffe üblichen Additive enthalten. Übliche Additive sind beispielsweise Gleitmittel wie Fettsäureester, deren Metallseifen, Fettsäureamide und Siliconverbindungen, Antiblockmittel, Inhibitoren, Stabilisatoren gegen Hydrolyse, Licht, Hitze und Verfärbung, Flammschutzmittel, Farbstoffe, Pigmente, anorganische oder organische Füllstoffe und Verstärkungsmittel. Verstärkungsmittel sind insbesondere faserartige Verstärkungsstoffe wie anorganische Fasern, die nach dem Stand der Technik hergestellt werden und auch mit einer Schlichte beaufschlagt sein können. Nähere Angaben über die genannten Hilfs- und Zusatzstoffe sind der Fachliteratur zu entnehmen, beispielsweise R.Gächter, H.Müller (Ed.): Taschenbuch der Kunststoff-Additive, 3.Ausgabe, Hanser Verlag, München 1989, oder DE-A 29 01 774.

Systematische Untersuchungen haben ergeben, daß eine homogene Verteilung der antimikrobiell wirksamen Substanz in der Polymermatrix notwendig ist, um die Wirkstoffdiffusion als einstellbaren Freisetzungsmechanismus nutzen zu können. Die antimikrobiell wirksame Substanz und das verwendete polymere Trägermaterial sollten daher eine hohe physiko-chemische Kompatibilität besitzen. Ein Maß für die Kompatibilität von Wirkstoff und Matrix ist die im System auftretende Grenzflächenenergie. Ist diese hoch, so sind Wirkstoff und Matrix wenig kompatibel und der Wirkstoff wird rasch abgegeben. Ist die Grenzflächenenergie zu niedrig, wird der Wirkstoff von der Polymermatrix stark gebunden; eine Freisetzung effektiver Mengen an der Oberfläche unterbleibt. Bei guter physikalisch-chemischer Kompatibilität von Wirkstoff und Matrix wird ein hoher Diffusionskoeffizient des Wirkstoffs im Polymer erzielt. Die Höhe der Abgaberate der antimikrobiell wirksamen Substanz kann in diesem Fall durch Variation der eingearbeiteten Wirkstoffmenge reguliert werden, da dann die freigesetzte Menge Wirkstoff der Konzentration in der Matrix proportional ist.

Zur Herstellung der erfindungsgemäßen wirkstoffhaltigen Polyetherblockamide werden Kombinationen von Matrix und Wirkstoff gewählt, die eine Grenzflächenenergie von 0.5 bis 30 mN/m, besonders bevorzugt 5 bis 15 mN/m aufweisen.

Die erfindungsgemäßen wirkstoffhaltigen Polyetherblockamide zeichnen sich dadurch aus, dass sie eine molekulardisperse Verteilung der antimikrobiell wirksamen Substanz in der polymeren Matrix aufweisen. Die hohe morphologische Homogenität der extrudierten wirkstoffhaltigen Kunststoffe konnte anhand licht- und rasterelektronenmikroskopischer Aufnahmen belegt werden. Zudem konnte mit Hilfe der rastermikroskopischen Aufnahmen demonstriert werden, dass das Polymer vor und nach der Freisetzung des eingearbeiteten Wirkstoffs eine glatte Oberfläche besitzt, d.h. die Biokompatibilität der Polymeroberfläche wird weder durch den Zusatz noch durch die Freisetzung des Wirkstoffs beeinträchtigt.

Auch die mechanischen Eigenschaften des Polymers werden durch den Zusatz der antimikrobiell wirksamen Substanzen in Mengen von 0,1 bis 10 Gew.-% nicht beeinträchtigt. Für bestimmte Werkstoff/Wirkstoffkombinationen wird sogar eine Verbesserung der mechanischen Eigenschaften beobachtet.

Vergleichbare wirkstoffhaltige Proben, die mittels der Gießfilmethode (solvent casting) hergestellt wurden, sind dagegen deutlich inhomogener. Rasterelektronenmikroskopische Untersuchungen belegen, daß die eingearbeiteten Wirkstoffe in der Polymermatrix und auf der Oberfäche z.T. in Form von Kristallverbänden vorliegen. Die Kristallverbände bewirken eine Verschlechterung der mechanischen Eigenschaften des Polymers. Zudem hinterlassen die herausgelösten Kristallverbände eine rauhe Oberfläche, was zu verminderter Biokompatibilität führt.

Die erfindungsgemäßen Formkörper können durch Extrudieren einer Schmelze bestehend aus dem Polymer und Wirkstoff hergestellt werden. Die Schmelze kann 0,01 bis 10 Gew-%, vorzugsweise 0,1 bis 5 Gew.-% Wirkstoff enthalten. Das Vermischen der Komponenten kann nach bekannten Techniken in jeglicher Weise erfolgen. Der Wirkstoff kann beispielsweise direkt in fester Form in die Polymerschmelze eingebracht werden. Es kann auch ein wirkstoffhaltiger Masterbatch direkt mit dem Polymer verschmolzen werden oder mit der bereits vorliegenden Polymerschmelze gemischt werden. Der Wirkstoff kann auch vor dem Schmelzen des Polymers mittels bekannter Techniken auf das Polymer aufgebracht werden (durch Tumbeln, Besprühen etc.)

Im übrigen kann die Vermischung/Homogenisierung der Komponenten nach bekannten Techniken über Kneter oder Schneckenmaschinen erfolgen, vorzugsweise in Ein- oder Doppelschneckenextrudern in einem Temperaturbereich zwischen 150 und 200°C.

Durch das Vermischen der Komponenten während des Extrusionsprozesses, wird eine homogene, molekulardisperse Verteilung des Wirkstoffs in der Polymermatrix erzielt, ohne daß zusätzliche Arbeitsschritte erforderlich wären.

Die Formgebung erfolgt durch die bekannten Techniken der Thermoplastverarbeitung (Spritzguß, Schlauchabzug etc.). Die Formkörper sind stippenfrei, flexibel, kleben nicht und können problemlos nach den gängigen Verfahren sterilisiert werden.

### Beispiele

### Beispiel 1 (Vergleich)

### Handelsübliches Polyetherblockamid: PEBAX® (Fa., Elf-Atochem, Philadelphia, PA 19103-3222)

### Beispiel 2

10 g Wirkstoff wurden in einem Intensivmischer auf 990 g wirkstofffreies PEBAX® aufgebracht Das wirkstoffhaltige Zylindergranulat wurde auf einem Zweiwellenextruder ZSK1 extrudiert. Es wurde eine klare Schmelze erhalten, die nach Abkühlung im Wasser/Luftbad und Stranggranulation ein farbloses, klares Zylindergranulat ergab.

Für mikrobiologische *in vitro* Untersuchungen sowie für die Bestimmung des Freisetzungsprofils des eingearbeiteten Wirkstoffs wurde das Granulat jeweils zu Prüfkörpern (Platten) spritzgegossen.

| **Beispiel** | **Eingearbeiteter Wirkstoff** |
|---|---|
| 2a | Kanamycin-Disulfat |
| 2b | Gentamicin-Sulfat |
| 2c | Ciprofloxacin-Betain |
| 2d | Doxycyclin-HCl |
| 2e | Clindamycin-HCl |
| 2f | Lincomycin-HCl |
| 2g | Fusidinsäure |
| 2h | Bacitracin |

### Beispiel 3

50 g Kanamycin-Disulfat wurden in einem Intensivmischer auf 950 g wirkstofffreies PEBAX® aufgebracht. Das wirkstoffhaltige Zylindergranulat wurde auf einem Zweiwellenextruder ZSK1 extrudiert. Es wurde eine klare Schmelze erhalten, die nach Abkühlung im Wasser/Luftbad und Stranggranulation ein farbloses, klares Zylindergranulat ergab.

### Beispiel 4

Zur Herstellung eines wirkstoffhaltigem Masterbatches wurden 380 g wirkstofffreies Zylindergranulat des Polyetherblockamids PEBAX® in einem geeigneten Lösungsmittel (z. B. Chloroform) gelöst und mit 20 g Kanamycin-Disulfat versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine klare Schmelze erhalten, die nach Abkühlung im Wasser/Luftbad und Stranggranulation ein farbloses, klares Zylindergranulat ergab.

### Beispiel 5

Zur Herstellung eines wirkstoffhaltigem Masterbatches wurden 380 g wirkstofffreies Zylindergranulat des Polyetherblockamids PEBAX® in einem geeigneten Lösungsmittel (z.B. Chloroform) gelöst und mit 20 g Gentamycin-Sulfat versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine klare Schmelze erhalten, die nach Abkühlung im Wasser/Luftbad und Stranggranulation ein farbloses, klares Zylindergranulat ergab.

### Beispiel 6

Zur Herstellung eines wirkstoffhaltigem Masterbatches wurden 380 g wirkstofffreies Zylindergranulat des Polyetherblockamids PEBAX® in einem geeigneten Lösungsmittel (z.B. Chloroform) gelöst und mit 20 g Bacitracin versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine klare Schmelze erhalten, die nach Abkühlung im Wasser/Luftbad und Stranggranulation ein farbloses, klares Zylindergranulat ergab.

### Beispiel 7

Aus den Prüfkörperplatten der in den Beispielen 1, 2a, 2b und 2h hergestellten Materialien wurden S2-Zugstäbe gestanzt und die Festigkeitskennwerte nach DIN 53 455 ermittelt. Die Zugverformungsreste der Prüfkörper wurden in Anlehnung an DIN 53 518 bestimmt. Die Bestimmung der E-Moduli erfolgte nach DIN 53 457.

Die Ergebnisse der Untersuchungen sind in Tabelle 1 zusammengefaßt. Sie dokumentieren, daß das thermisch-mechanische Eigenschaftsniveau von ungefülltem PEBAX® durch Zusatz von antimikrobiell wirksamen Substanzen im Rahmen der Meßgenauigkeiten nicht signifikant verändert wird.

**Tabelle 1:**

| Thermisch-mechanisches Eigenschaftsniveau von ungefülltem PEBAX® und von wirkstoffhaltigen PEBAX®-Mustern. | | | | |
|---|---|---|---|---|
| **Meßwerte** | **Beispiel 1** PEBAX® | **Beispiel 2a** PEBAX® + 1 Gew.% Kanamycin | **Beispiel 2b** PEBAX® + 1 Gew.% Gentamicin | **Beispiel 2h** PEBAX® + 1 Gew.% Bacitracin |
| Zugfestigkeit [MPa] | 22.0 | 20.3 | 21.0 | 22.3 |
| Bruchdehnung [%] | 1080 | 995 | 1058 | 1073 |
| Bleibende Dehnung bei 200% Dehnung | 46 | 49 | 45 | 46 |
| E'(10 °C) [MPa] | 79 | 107 | 114 | 89 |
| E'(36 °C) [MPa] | 24 | 35 | 40 | 27 |

### Beispiel 8

Die Ermittlung der Freisetzungsprofile von wirkstoffhaltigen Polymerproben erfolgte durch Elution in Millipore Wasser (0,1 % NaN₃). In einem typischen Experiment wurden dazu 5 g wirkstoffhaltige Polymerplättchen aus PEBAX® (Fläche: 1 cm²) bei 37°C mit 20 ml Millipore Wasser versetzt und mit konstanter Geschwindigkeit gerührt. In regelmäßigen Zeitabständen von 24 h wurde das Elutionsmittels für die quantitative Bestimmung des Wirkstoffgehaltes entnommen und durch frisches Millipore Wasser ersetzt. Die Quantifizierung des freigesetzten Wirkstoffs in den entsprechenden Lösungen erfolgte via HPLC-Analytik. Alle Versuchsserien wurden 2-fach durchgeführt, die quantitative Ermittlung des Wirkstoffgehaltes erfolgte jeweils als Doppelbestimmung.

Die Ergebnisse der Untersuchungen sind in Tabelle 2 zusammengefaßt. Sie dokumentieren, daß die wirkstoffhaltigen Polymeren über einen längeren Zeitraum (2 Wochen) kontinuierlich die entsprechende antimikrobiell wirksame Substanz an der Oberfläche freisetzen. Zudem konnte gezeigt werden, daß die Wirkstoffdiffusion aus dem Polymer als einstellbarer Freisetzungsmechanismus genutzt werden kann: Je höher die eingearbeitete Wirkstoffmenge, desto größer ist die aus der Polymermatrix freigesetzte Wirkstoffkonzentration im Elutionsmedium.

**Tabelle 2:**

| Freisetzungsprofile wirkstoffhaltiger Polymerproben. Es ist jeweils die Konzentration [mg/l] des aus der Polymerprobe freigesetzten Wirkstoffs angegeben. **Beispiel 1:** Kontrollprobe wirkstofffreies PEBAX®, **Beispiel 2c:** 1,0 Gew.-% Ciprofloxacin-Betain in PEBAX®, **Beispiel 2d:** 1,0 Gew.-% Doxycyclin-HCl in PEBAX®, **Beispiel 2e:** 1,0 Gew.-% Clindamycin-HCl in PEBAX®, **Beispiel 2f:** 1,0 Gew.-% Lincomycin-HCl in PEBAX®, **Beispiel 2g:** 1,0 Gew.-% Fusidinsäure in PEBAX® | | | | | | | | | | |
|---|---|---|---|---|---|---|---|---|---|---|
| **Zeit [h]** | **0** | **22** | **45** | **69** | **93** | **165** | **189** | **213** | **237** | **261** |
| Beispiel 1 | 0 | 0 | 0 | 0 | 0 | 0 | 0 | 0 | 0 | 0 |
| Beispiel 2c | 0 | 88,13 | 135,2 | 183,5 | 230,5 | 279,1 | 321,0 | 353,7 | 387,0 | 417,5 |
| Beispiel 2d | 0 | 537,77 | 730,2 | 834,8 | 898,4 | 995,1 | 1032,4 | 1052,9 | 1069,5 | 1085,2 |
| Beispiel 2e | 0 | 331,61 | 453,0 | 540,2 | 603,8 | 702,7 | 746,2 | 781,7 | 816,0 | 847,1 |
| Beispiel 2f | 0 | 286,01 | 371,4 | 427,3 | 463,8 | 540,1 | 566, | 588,4 | 610,5 | 630,5 |
| Beispiel 2g | 0 | 15,1 | 20,4 | 27,0 | 34,5 | 42,5 | 52,0 | 61,6 | 70,8 | 80,5 |

### Beispiel 9

Die Prüfung der antimikrobiellen Wirksamkeit der modifizierten Polymeren erfolgte an den Bakterienstämmen *S. epidermidis* 0-47- und *S. epidermidis* 0-47+ (Fa. bioMerieux, D-72622 Nürtingen) sowie den Teststämmen *S. aureus* ATCC 25923, *S. epidermidis* ATCC 14990 und *S. saproph.* ATCC 43867.

Die Bakterienstämme wurden jeweils in einer Übernachtkultur auf Standard II-Nähragar (Fa. Merck KGaA, D-64293 Darmstadt) kultiviert und in NaCl-Lösung (0,85 %) suspendiert. Die erhaltene Bakteriensuspension mit einer Dichte von 0.5 MacFarland wurde 1:100 in NaCl-Lösung (0,85 %) verdünnt und auf Agarplatten aufgebracht (Mueller-Hinton Agar, Fa. Merck KGaA, D-64293 Darmstadt). Die 1 cm² großen Polymerproben wurden sterilisiert, unter leichtem Druck auf die Agarplatten gelegt und 20 Stunden bei 37°C inkubiert. Nach der Inkubation wurden die Agarplatten auf Hemmhöfe kontrolliert und die Hemmhöfe ausgemessen.

Es wurden 21 Versuchsserien (3 unterschiedliche Polymermuster gegenüber 7 Teststämmen) durchgeführt. Die Ergebnisse des Agardiffusionstests sind in Tabelle 3 zusammengefaßt. Sie zeigen, daß um die wirkstoffhaltigen Polymermuster im Vergleich zu der wirkstofffreien Probe eine Hemmzone ausgebildet wurde, in der kein Bakterienwachstum stattfindet, d.h. die wirkstoffhaltigen Polymerproben weisen eine substantielle antimikrobielle Wirkung gegenüber den verwendeten Teststämmen auf.

### Tabelle 3:

Prüfung der antimikrobiellen Wirksamkeit wirkstofffreier und wirkstoffhaltiger Polymermuster gegenüber verschiedenen Teststämmen im Agardiffusionstest. Die antimikrobielle Wirkung wird durch Ausbildung eines Hemmhofes angezeigt. Die Hemmhofdurchmesser sind in *mm* angegeben.

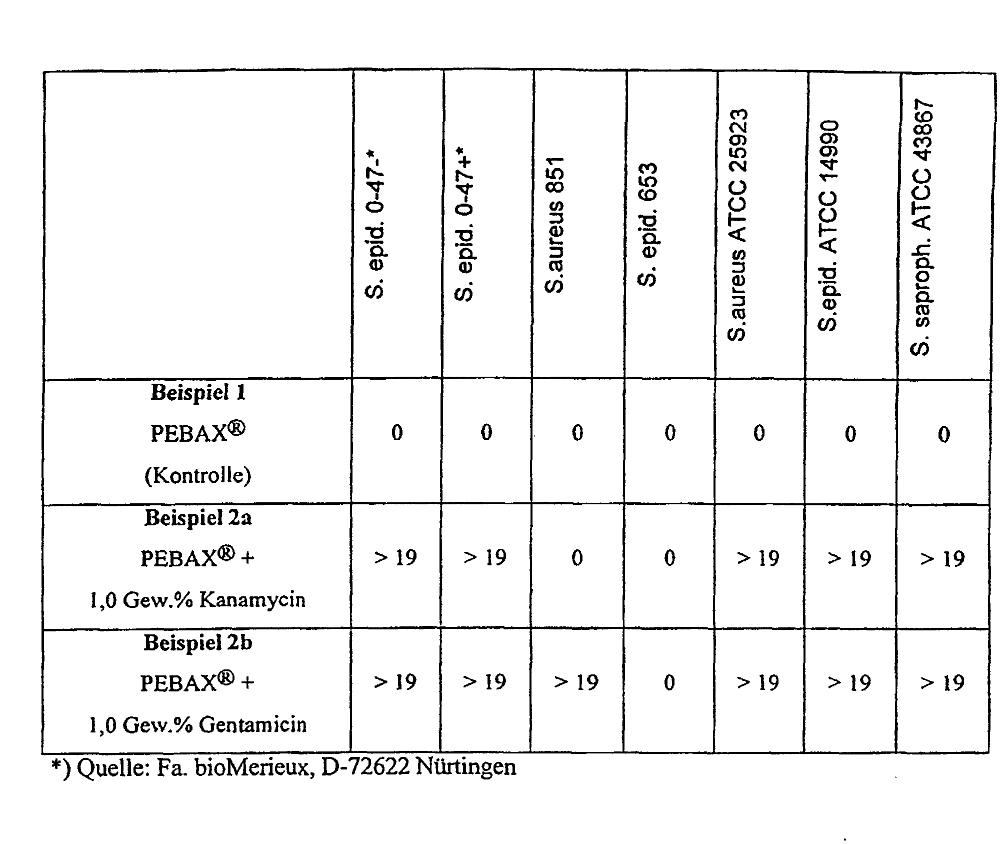

## Patentansprüche

1. Polyetherblockamide aus Polymerketten, die aus wiederkehrenden Einheiten entsprechend der Formel I aufgebaut sind 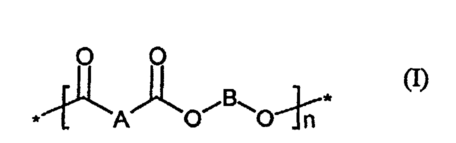 in welcher
A die von einem Polyamid mit 2 Carboxylendgruppen durch Verlust der letzteren abgeleitete Polyamidkette ist und
B die von einem Polyoxyalkylenglycol mit endständigen OH-Gruppen durch Verlust der letzteren abgeleitete Polyoxyalkylenglycolkette ist und
n die Zahl der die Polymerkette bildenden Einheiten ist
und die antimikrobiell wirksame Stoffe in homogener Verteilung enthalten aus der Gruppe bestehend aus
a) Nalixidinsäure, Pipemidsäure, Cinoxacin, Ciprofloxacin, Norfloxacin, Ofloxacin, Pefloxacin, Enoxacin,
b) Aminoglycoside,
c) Makrolide,
d) Polypeptide,
e) Lincomycine,
f) antimycobakterielle Mittel,
g) Fusidinsäure,
wobei die Kombination des Polyetherblockamids und der antimikrobiell wirksamen Stoffe eine Grenzflächenenergie von 0,5 bis 30 mN/m aufweisen,
für die Herstellung von medizinischen Formkörpern für Kurzzeitimplantate, die geeignet sind, für einen längeren Zeitraum eine Oberflächenbesiedlung durch Keime zu verhindern.

2. Verfahren zur Herstellung der antimikrobiell wirksame Stoffe in homogener Verteilung enthaltenden Polyetherblockamide gemäß Anspruch 1 durch homogenes Vermischen eines antimikrobiell wirksamen Stoffs und eines Polyetherblockamids.

3. Formkörper aus antimikrobiell wirksame Stoffe in homogener Verteilung enthaltenden Polyetherblockamiden.

4. Verwendung der antimikrobiell wirksame Stoffe in homogener Verteilung enthaltenden Polyetherblockamide gemäß Anspruch 1 in medizinischen Artikeln.

## Claims

1. Polyether block amides comprising polymer chains synthesised from repeating units corresponding to formula I 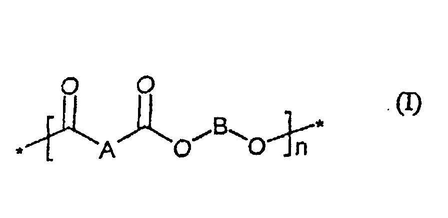 in which
A is the polyamide chain derived from a polyamide having 2 carboxyl terminal groups by loss of the latter and
B is the polyoxyalkylene glycol chain derived from a polyoxyalkylene glycol having terminal OH groups by loss of the latter and
n is the number of units forming the polymer chain
and containing antimicrobially active substances in a homogeneous distribution from the group consisting of
a) nalixidic acid, pipemidic acid, cinoxacin, ciprofloxacin, norfloxacin, ofloxacin, perfloxacin, enoxacin,
b) aminoglycosides,
c) macrolides,
d) polypeptides,
e) lincomycins,
f) antimycobacterial agents,
g) fusidic acid,
whereby the combination of the polyether block amide and the antimicrobially active substances displays an interfacial energy of 0.5 to 30 mN/m,
for the production of medical mouldings for temporary implants, which are suitable for preventing surface colonisation by microbes over an extended period.

2. Process for producing the polyether block amides containing antimicrobially active substances in a homogeneous distribution according to claim 1 by the homogeneous mixing of an antimicrobially active substance and a polyether block amide.

3. Mouldings made from polyether block amides containing antimicrobially active substances in a homogeneous distribution.

4. Use of the polyether block amides containing antimicrobially active substances in a homogeneous distribution according to claim 1 in medical articles.

## Revendications

1. Polyéther-polyamide séquencé consistant en chaînes polymères constituées de motifs répétés répondant à la formule I 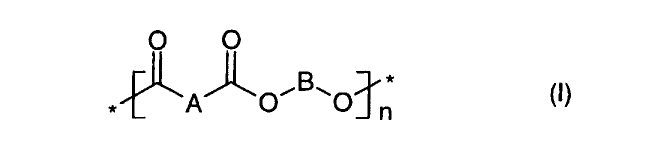 dans laquelle
A représente la chaîne de polyamide dérivant d'un polyamide à 2 groupes terminaux carboxyle par perte de ces derniers et
B représente la chaîne de polyoxyalkylèneglycol dérivant d'un polyoxyalkylèneglycol à groupes OH terminaux par perte de ces derniers et
n est le nombre des motifs formant la chaîne polymère,
et contiennent en répartition homogène une substance à activité antimicrobienne choisie dans le groupe consistant en
a) l'acide Nalixidinique, l'acide Pipémidique, la Cinoxacine, la Ciprofloxacine, la Norfloxacine, l'Ofloxacine, la Pefloxacine, l'Enoxacine,
b) des aminoglycosides,
c) des macrolides
d) des polypeptides,
e) des lincomycines,
f) des agents antimycobactériens,
g) l'acide Fusidique,
la combinaison du polyéther-polyamide séquencé et de la substance à activité antimiccrobienne présentant une énergie interfaciale de 0,5 à 30 mN/m,
pour la fabrication d'objets moulés médicinaux pour implants de courte durée propres à éviter pendant une durée prolongée une colonisation de la surface par des germes.

2. Procédé pour la préparation de polyéther-polyamides séquencés contenant en répartition homogène des substances à activité antimicrobienne selon la revendication 1 par mélange homogène d'une substance à activité antimicrobienne et d'un polyéther-polyamide séquencé.

3. Objets moulés consistant en polyéther-polyamides séquencés contenant en répartition homogène des substances à activité antimicrobienne.

4. Utilisation des polyéther-polyamides séquencés contenant en répartition homogène des substances à activité antimicrobienne selon la revendication 1 dans des articles médicinaux.
